Europäisches Patentamt

European Patent Office

Office européen des brevets

(19)

(11) Veröffentlichungsnummer: **0 001 236**
**B1**

(12) **EUROPÄISCHE PATENTSCHRIFT**

(21) Anmeldenummer: **78100813.1**

(22) Anmeldetag: **04.09.78**

(51) Int. Cl.³: **C 07 C 113/04,**
**G 01 N 27/46**

(54) Verfahren zur kontinuierlichen indirekten Diazotierung von aromatischen Aminen

(30) Priorität: **17.09.77 DE 2741925**

(43) Veröffentlichungstag der Anmeldung:
**04.04.79 Patentblatt 79/07**

(45) Bekanntmachung des Hinweises auf die Patenterteilung:
**01.10.80 Patentblatt 80/20**

(84) Benannte Vertragsstaaten:
**CH DE FR GB**

(56) Entgegenhaltungen:
**DE - C - 960 205**
**FR - A - 2 309 517**
**FR - A - 2 320 937**

(73) Patentinhaber: **BAYER AG**
**Zentralbereich Patente, Marken und Lizenzen**
**D - 5090 Leverkusen 1, Bayerwerk (DE)**

(72) Erfinder: **Breig, Kurt, Dr.**
**Leopold-Gmelin-Strasse 80**
**D - 5000 Köln 80 (DE)**
**Dehmel, Georg**
**Jakob-Böhme-Strasse 6**
**D - 5000 Köln 80 (DE)**
**Hamm, Norbert**
**Max-Planck-Strasse 63**
**D - 5072 Schildgen (DE)**

## Verfahren zur kontinuierlichen indirekten Diazotierung von aromatischen Aminen

Die Erfindung betrifft ein Verfahren zur kontinuierlichen indirekten Diazotierung von aromatischen Aminen, insbesondere von schwach basischen, beispielsweise Nitrogruppen enthaltenden Aminen, wobei das Amin mit Wasser und einem Überschuß an Nitrit vermischt wird und dieses Gemisch in einen Diazotierbehälter, wo es mit einer Mineralsäure reagiert, gepumpt wird.

Die kontinuierliche Diazotierung ist beispielsweise in der DE—AS 12 31 251 beschrieben. Eine wäßrige Amin- oder eine mineralsaure Aminsalzsuspension läuft gleichzeitig mit Nitritlösung und Mineralsäure in den Diazotierbehälter ein. Insbesondere bei schwach basischen Aminen hat die beschriebene kontinuierliche Fahrweise den Nachteil, daß bei Konzentrationsschwankungen in den Ausgangsprodukten abwechselnd Nitritüberschuß und -unterschuß vorherrscht und bei einem Nitritunterschuß die nicht sofort diazotierten Aminanteile mit der Diazoverbindung zu einer Diazoaminoverbindung reagieren. Durch diese Nebenreaktion wird die Qualität und die Ausbeute des daraus gebildeten Farbstoffes bzw. Zwischenproduktes herabgesetzt.

Nach dem erfindungsgemäß Verfahren wird eine Verbesserung der Produktqualität bei kontinuierlicher Fahrweise erreicht, wenn ein Teilstrom von einer Vorlage zu dem Diazotierbehälter durch einen Analysator, in dem die Verweilzeit mindestens um einen Faktor 3 bis 4 kleiner ist als in dem Diazotierbehälter, geleitet wird, und wo im Analysator mit einer elektrochemischen Meßmethode festgestellt wird, wenn für das betreffende Amin der gewünschte Nitritüberschuß über- bzw. unterschritten wird und dann die Zufuhr von Nitrit in die Vorlage so lange gestoppt bzw. fortgeführt wird, bis im Analysator der gewünschte Nitritüberschuß wieder erreicht ist. Gegenstand der Erfindung ist also ein kontinuierliches Verfahren zur indirekten Diazotierung, bei dem der gewünschte Nitritüberschuß aufgrund einer elektro-chemischen Meßmethode nicht erst im eigentlichen Diazotiergefäß, sondern bereits in der Vorlage bei einem pH-Wert eingestellt wird, bei dem noch keine Diazotierungsreaktion erfolgt. Es wird so eine störungsfreie "indirekte Diazotierung" im Diazotiergefäß erreicht, da das Amin bereits mit der erwünschten Nitritkonzentration in die Mineralsäure eindosiert werden kann. Der im Teilstrom zwischen Vorlage und Diazotiergefäß geschaltete Analysator reagiert leicht auf zeitliche Schwankungen der Aminkonzentration in der Vorlage, die somit Gesamtreaktion im Diazotiergefäß nicht stören können.

Der Begriff indirekte Diazotierung wird verwendet für eine Fahrweise, bei der das Amin erst mit Nitritüberschuß versetzt und dann einer Mineralsäure zugeführt wird.

Das Verfahren wird bevorzugt so betrieben, daß der gewünschte Nitritüberschuß im Analysator einem Nitritüberschuß in der Vorlage von 1 bis 10%, vorzugsweise 2%, entspricht.

Besonders bevorzugt ist ein Verfahren, bei dem der gewünschte Nitritüberschuß potentiometrisch, voltametrisch oder polarographisch ermittelt wird.

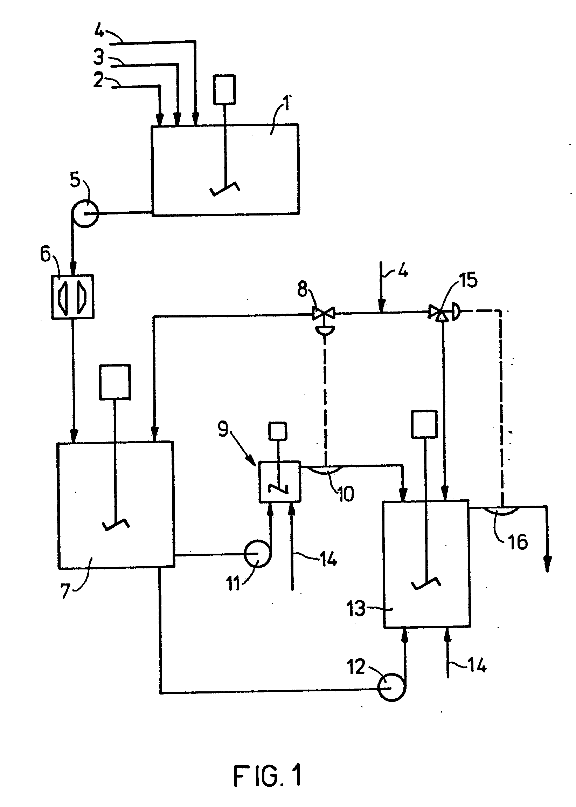

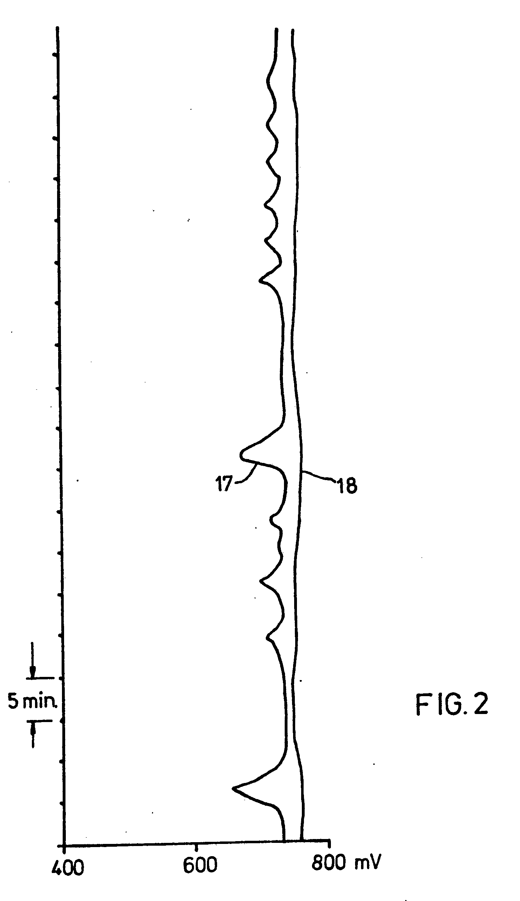

Das Verfahren ist im folgenden beispielhaft beschrieben und schematisch in Figur 1 dargestellt. Figur 2 zeigt Potentialschwankungen im Analysator und im Diazotierbehälter.

In einer Vorlage 1 wird das zu diazotierende Amin 2 mit Wasser 3 und Nitrit 4 vermischt. Die Nitritmenge in 1 soll etwa 50 bis 95%, vorzugsweise 90%, der erforderlichen Nitritmenge sein. Ob diese Vorlage 1 kontinuierlich über Dosiereinrichtungen oder diskontinuierlich beschickt wird, ist unerheblich. Die angemaischte Aminsuspension wird von einer Pumpe 5 gegebenenfalls durch eine Zerkleinerungsmaschine 6 in die Vorlage 7 gefördert. Eine Zerkleinerungsmaschine 6 ist besonders bei grobkristallinen Aminen erforderlich; geeignet sind Zahnkolloidmühlen, Korundscheibenmühlen oder Perlmühlen. In die Vorlage 7 wird weiteres Nitrit 4 über ein Ventil 8 zugegeben. Nitrit 4 soll in 7 im Überschuß vorhanden sein, überlicherweise beträgt der Überschuß 1 bis 10% der stöchiometrisch erforderlichen Nitritmenge, vorzugsweise 2%. Die Zugabe von Nitrit 4 zu 7 wird von einem Analysator 9 aus gesteuert. Der Analysator 9 mißt mit elektrochemischen Meßmethoden 10 (Redoxpotentiometrie, Voltametrie oder Polarographie) die Nitritkonzentration der aus der Vorlage 7 ablaufenden Aminsuspension oder -lösung. Er ist so eingestellt, daß bei Nitritunterschuß über 8 der Vorlage 7 Nitrit 4 zugeführt wird, und bei Erreichen des gewünschten Überschusses in 10 die Zugabe von 4 über 8 gestoppt wird. Durch den Analysator 9 wird üblicherweise nur ein relativ kleiner Mengenstrom 11 gepumpt. Die Hauptmenge der Aminsuspension aus 7 wird über die Pumpe 12 in den Diazotierbehälter 13 gefördert. Durch Zugabe von Mineralsäure 14 zu 9' und 13 wird die Aminsuspension quantitativ diazotiert. Als zusätzliche Kontrollmöglichkeit ist am Ablauf von 13 eine elektrochemische Meßvorrichtung 16, ähnlich aufgebaut wie 10, vorhanden, mit der auch dort der Nitritüberschuß bestimmt wird. Nur in Ausnahmefällen wird durch 16 eine Nitritzugabe 4 über das Ventil 15 in den Behälter 13 veranlaßt; beispielsweise beim Anfahren kann dieser Fall eintreten.

Die Verweilzeit in dem Diazotierbehälter 13 muß auf das Amin abgestimmt sein. Die Verweilzeit ist definiert als Quotient des aktiven Volumens des Behälters und des Volumenflusses pro Zeiteinheit. Die Verweilzeit liegt in den meisten Fällen zwischen 2 und 10 Minuten.

Im Vergleich zur Verweilzeit in 13 muß die Verweilzeit in 9 mindestens um einen Faktor 3 kleiner sein als in 13. Es ist überraschend, daß bereits ein Faktor 3 oder 4 zwischen der Verweilzeit in 9 und 13 ausreicht, um den gewünschten Nitritüberschuß in 13 konstant zu halten.

In der Figur 2 ist ein typisches Schreiber-Diagramm von den Signalen 17, 18, die von 10 und 16 ausgehen, dargestellt. In diesem Beispiel wird in 10 und 16 das Redox-Potential gemessen. Beide Meßstellen sind auf gleiche Empfindlichkeit eingestellt. Im Analysator 9 schwankt das Potential erheblich; in dem Diazotierbehälter 13 wird durch die erfindungsgemäße Betriebsweise ein nahezu konstanter Nitritüberschuß gehalten.

Beispiel

*Diazotierung von 2-Nitro-4-methyl-anilin*

In der Vorlage 1 werden 1670 Teile 2-Nitro-4-methyl-anilin in 2300 Teilen Wasser und 2300 Teilen Natriumnitrit-Lösung (30 g/100 ml) suspendiert. Die Nitritmenge entspricht 90% der theoretisch für die vollständige Diazotierung erforderlichen Menge. Über eine Korundscheibenmühle 6 gelangt die Suspension in die Vorlage 7. Der Mengenstrom durch den Analysator 9, der ein Volumen von 0,2 1 hat, beträgt 5,2 1/h. Die Verweilzeit beträgt 2,3 Minuten. Auch im Betriebsmaßstab ist ein Analysator dieser Größe gut geeignet. In einer Versuchsanlage wird jedoch der Hauptstrom über die Pumpe 12 erheblich kleiner ausgelegt als er im Produktionsbetrieb wäre, so daß man zur Simulation im Versuchsbetrieb diesen Strom entsprechend herabsetzen muß, um die gewünschten Verweilzeiten in den Behältern zu erreichen. Die Verweilzeit in dem Behälter 13 beträgt etwa 7 Minuten. Der Nitritüberschuß an den Meßstellen 10 und 16 wird durch eine Redoxpotentialmessung festgestellt. Die Meßstellen sind mit Platin und Silber/Silberchlorid-Elektroden ausgestattet. In die Behälter 9 und 13 wird 35%ige Salzsäure eingepumpt. Die Behälter 7, 9 und 13 werden in diesem Beispiel durch Solekühlung auf temperaturen von 5, 30 und 20°C gehalten. Die aus dem Diazotierbehälter 13 auslaufende Diazotierung wird nach Filtration mit A-Kohle direkt weiterverarbeitet.

In Figur 2 sind die geschriebenen Anzeigen 17 und 18 von 10 und 16 dargestellt.

In gleicher Weise lassen sich andere aromatische Amine kontinuierlich diazotieren, wie z.B. 2-Chlor-4-nitroanilin, 4-Chlor-2-nitranilin, 2-Methoxy-4-nitranilin, 3,3'-Dichlorbenzidin, 2,5-Dichlor-4-sulfanilsäure, Anilin-mono- und -disulfonsäuren, Aminonaphthalin-sulfonsäuren, p-Nitranilin, Dichloranilin, 1-Amino-2-hydroxy-5-chlorbenzolsulfonsäure-(3).

Patentanspruch

1. Verfahren zur kontinuierlichen indirekten Diazotierung von aromatischen Aminen, insbesondere von schwach basischen, beispielsweise Nitrogruppen enthaltenden Aminen, wobei das Amin mit Wasser und einem Überschuß an Nitrit vermischt wird und dieses Gemisch in einen Diazotierbehälter, wo es mit einer Mineralsäure reagiert, gepumpt wird, dadurch gekennzeichnet, daß ein Teilstrom von einer Vorlage (7) zu dem Diazotierbehälter (13) durch einen Analysator (9), in dem die Verweilzeit mindestens um den Faktor 3 bis 4 kleiner ist als in dem Diazotierbehälter (13), geleitet wird, und wo im Analysator (9) mit einer elektrochemischen Meßmethode (10) festgestellt wird, wenn für das betreffende Amin der gewünschte Nitritüberschuß über-bzw. unterschritten wird und dann die Zufuhr von Nitrit (4) in die Vorlage (7) so lange gestoppt bzw. fortgeführt wird, bis im Analysator (9) der gewünschte Nitritüberschuß wieder erreicht ist.

2. Verfahren nach Anspruch 1, dadurch gekennzeichnet, daß der gewünschte Nitritüberschuß im Analysator (9) einem Nitritüberschuß in der Vorlage (7) von 1 bis 10%, vorzugsweise 2%, entspricht.

3. Verfahren nach Ansprüchen 1 und 2, dadurch gekennzeichnet, daß der gewünschte Nitritüberschuß potentiometrisch, voltametrisch oder polarographisch ermittelt wird.

Claims

1. A process for the continuous indirect diazotization of aromatic amines, in particular of weakly basic amines, for example amines containing nitro groups, wherein the amine is mixed with water and an excess of nitrite and this mixture is pumped into a diazotization vessel in which it reacts with a mineral acid, characterized in that a component stream is led from a receiver (7) to the diazotization vessel (13) through an analyzer (9), the residence time of the component stream in the analyzer being shorter by a factor of at least 3 to 4 than the residence time of the mixture in the diazotization vessel, and wherein the point in time at which the nitrite excess for the particular amine exceeds or falls below the required level is determined in the analyzer (9) by an electrochemical measuring technique and then the introduction of nitrite (4) into the receiver (7) is stopped or continued until the required nitrite excess is again reached in the analyzer (9).

2. A process according to claim 1, characterized in that the required nitrite excess in the analyzer corresponds to a nitrite excess in the receiver (7) of 1% to 10%, preferably 2%.

3. A process according to claims 1 and 2, characterized in that the required nitrite excess is determined potentiometrically, voltametrically or polarographically.

**Revendications**

1. Procédé de diazotation indirecte continue d'amines aromatiques, notamment d'amines faiblement basiques portant par exemple des groupes nitro, dans lequel l'amine est mélangée avec de l'eau et un excès de nitrite et ce mélange est introduit par pompage dans un récipient de diazotation où il réagit avec un acid minéral, caractérise par le fait qu'un courant partiel est transféré d'un appareil (7) dans le récipient (13) de diazotation en passant par un analyseur (9) dans lequel la durée de séjour est au moins trois à quatre fois plus courte que dans l'appareil (13) de diazotation, et une méthode électrochimique de mesure (10) détermine dans l'analyseur (9) le moment où l'excès désiré de nitrite est dépassé ou est insuffisant pour l'amine en question, puis l'arrivée de nitrite (4) dans l'appareil (7) est arrêtée ou, respectivement, poursuivie jusqu'à ce que l'excès désiré de nitrite ait été rétabli dans l'analyseur (9).

2. Procédé suivant la revendication 1, caractérisé en ce que l'excès désiré de nitrite dans l'analyseur (9) correspond à un excès de nitrite dans l'appareil (7) de 1 à 10%, de préférence 2%.

3. Procédé suivant l'une des revendications 1 et 2, caractérisé en ce que l'excès désiré de nitrite est déterminé par une mesure potentiométrique, voltamétrique ou polarographique.

FIG. 1

FIG. 2